(19) Europäisches Patentamt
European Patent Office
Office européen des brevets

(11) **EP 4 648 019 A1**

(12) **EUROPEAN PATENT APPLICATION**

(43) Date of publication:
**12.11.2025 Bulletin 2025/46**

(21) Application number: **25173979.3**

(22) Date of filing: **02.05.2025**

(51) International Patent Classification (IPC):
***G06V 10/25*** (2022.01)        ***G06V 10/20*** (2022.01)
***G06V 40/16*** (2022.01)

(52) Cooperative Patent Classification (CPC):
**G06V 10/25; G06V 10/255; G06V 40/162**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **09.05.2024 US 202418660187**

(71) Applicant: **Avago Technologies International
Sales
Pte. Limited
Singapore 768923 (SG)**

(72) Inventors:
• **Kishore, Chhavi**
**560102 Bangalore, KA (IN)**
• **Wyman, Richard Hayden**
**Sunnyvale, CA, 94085 (US)**
• **Berbecel, Gheorghe**
**Irvine, CA, 92614 (US)**

(74) Representative: **Grünecker Patent- und
Rechtsanwälte
PartG mbB
Leopoldstraße 4
80802 München (DE)**

(54) **METHODS AND SYSTEMS FOR IMAGE AND VIDEO PROCESSING USING SKIN DETECTION**

(57)    The subject technology is directed to methods and systems for enhancing skin detection in video and image processing. According to an embodiment, the subject technology provides a method that includes receiving an image comprising a first region. The method further includes generating a first confidence value using a first model. A first confidence value is associated with a first pixel of the first region. The first confidence value is associated with a first probability of the first region being a skin region. Subsequent image processing is performed based at least on the first confidence value, enabling dynamic adjustments that enhance the accuracy and visual quality of skin detection in diverse imaging environments. There are other embodiments as well.

*200*

Figure 2

## Description

### FIELD OF INVENTION

The present invention is directed to image and video processing systems and methods.

### BACKGROUND OF THE INVENTION

In the realm of digital image and video processing, enhancing the visual quality of human skin areas in multimedia content presents a significant challenge. The human visual system (HVS) is particularly sensitive to imperfections in facial regions, making the accurate rendering of these areas essential for the overall perception of image quality. Some approaches utilize color models to detect skin regions by applying predefined color thresholds. However, these approaches often fail to adequately differentiate between skin and non-skin regions due to their uniform application of adjustments across all areas. Such indiscriminate processing can lead to over-processing or under-processing of skin tones, detracting from the realism and fidelity of human portrayals. Additionally, these methods rarely cater to the specific needs of individuals with color vision deficiencies (CVD), who require tailored color adjustments to enhance visual clarity and color distinction.

Various approaches for enhancing skin detection in image and video processing have been explored, but they have proven to be insufficient. New and improved methods and systems are desired.

### BRIEF DESCRIPTION OF THE DRAWINGS

Figure 1 is a simplified diagram illustrating a computing device for video and image processing according to embodiments of the subject technology.

Figure 2 is a simplified diagram illustrating a system for video and image processing according to embodiments of the subject technology.

Figure 3 is a simplified flow diagram illustrating a method for video and image processing according to embodiments of the subject technology.

### DETAILED DESCRIPTION OF THE INVENTION

The subject technology is directed to methods and systems for enhancing skin detection in video and image processing. According to an embodiment, the subject technology provides a method that includes receiving an image comprising a first region. The method further includes generating a first confidence value using a first model. A first confidence value is associated with a first pixel of the first region. The first confidence value is associated with a first probability of the first region being a skin region. Subsequent image processing is performed based at least on the first confidence value, enabling dynamic adjustments that enhance the accuracy and visual quality of skin detection in diverse imaging environments. There are other embodiments as well.

As mentioned above, existing methods for skin detection in image and video processing are inadequate. For example, some approaches rely on fixed threshold values within specific color models, which can lead to inconsistent results when faced with complex imaging scenarios such as mixed lighting or rapid scene changes. This may result in inaccurate skin detection, particularly under non-ideal lighting conditions or with diverse skin colors. Additionally, such approaches involve applying uniform adjustments across the entire image, which can lead to harsh edges or unrealistic smoothing effects. Furthermore, various techniques fail to account for the different color perception needs of users with color vision deficiencies, further limiting their applicability.

In various embodiments, the subject technology provides methods and systems that enhance the accuracy and adaptability of skin detection in video and image processing. For instance, it adopts multiple color models, each targeting different characteristics of skin tones. This multi-model approach enhances the system's ability to detect skin regions across a broad range of imaging conditions and skin colors. Furthermore, embodiments of the subject technology involve an algorithmic framework that integrates outputs from these color models to generate a skin confidence map, which quantifies the likelihood of each pixel belonging to a skin region based on combined data points. Such an approach addresses the challenges posed by varying imaging conditions by allowing for refined adjustments to image processing techniques, such as adaptive smoothing and edge enhancement, which are tailored based on the confidence levels derived from the map. By streamlining the image processing workflow, the subject technology reduces the complexity and time involved in post-processing phases, enabling more rapid production cycles for digital media content.

The following description is presented to enable one of ordinary skill in the art to make and use the invention and to incorporate it in the context of particular applications. Various modifications, as well as a variety of uses in different applications, will be readily apparent to those skilled in the art, and the general principles defined herein may be applied to a wide range of embodiments. Thus, the present invention is not intended to be limited to the embodiments presented but is to be accorded the widest scope consistent with the principles and novel features disclosed herein.

In the following detailed description, numerous specific details are set forth in order to provide a more thorough understanding of the present invention. However, it will be apparent to one skilled in the art that the present invention may be practiced without necessarily being limited to these specific details. In other instances, well-known structures and devices are shown in block diagram form, rather than in detail, in order to avoid obscuring the present invention.

The reader's attention is directed to all papers and documents which are filed concurrently with this specification and which are open to public inspection with this specification, and the contents of all such papers and documents are incorporated herein by reference. All the features disclosed in this specification, (including any accompanying claims, abstract, and drawings) may be replaced by alternative features serving the same, equivalent, or similar purpose unless expressly stated otherwise. Thus, unless expressly stated otherwise, each feature disclosed is one example only of a generic series of equivalent or similar features.

Furthermore, any element in a claim that does not explicitly state "means for" performing a specified function, or "step for" performing a specific function, is not to be interpreted as a "means" or "step" clause as specified in 35 U.S.C. Section 112, Paragraph 6. In particular, the use of "step of" or "act of" in the Claims herein is not intended to invoke the provisions of 35 U.S.C. 112, Paragraph 6.

When an element is referred to herein as being "connected" or "coupled" to another element, it is to be understood that the elements can be directly connected to the other element, or have intervening elements present between the elements. In contrast, when an element is referred to as being "directly connected" or "directly coupled" to another element, it should be understood that no intervening elements are present in the "direct" connection between the elements. However, the existence of a direct connection does not exclude other connections, in which intervening elements may be present.

Moreover, the terms left, right, front, back, top, bottom, forward, reverse, clockwise, and counterclockwise are used for purposes of explanation only and are not limited to any fixed direction or orientation. Rather, they are used merely to indicate relative locations and/or directions between various parts of an object and/or components.

Furthermore, the methods and processes described herein may be described in a particular order for ease of description. However, it should be understood that, unless the context dictates otherwise, intervening processes may take place before and/or after any portion of the described process, and further various procedures may be reordered, added, and/or omitted in accordance with various embodiments.

Unless otherwise indicated, all numbers used herein to express quantities, dimensions, and so forth should be understood as being modified in all instances by the term "about." In this application, the use of the singular includes the plural unless specifically stated otherwise, and the use of the terms "and" and "or" means "and/or" unless otherwise indicated. Moreover, the use of the terms "including" and "having," as well as other forms, such as "includes," "included," "has," "have," and "had," should be considered non-exclusive. Also, terms such as "element" or "component" encompass both elements and components comprising one unit and elements and components that comprise more than one unit, unless specifically stated otherwise.

As used herein, the phrase "at least one of" preceding a series of items, with the term "and" or "or" to separate any of the items, modifies the list as a whole, rather than each member of the list (i.e., each item). The phrase "at least one of" does not require the selection of at least one of each item listed; rather, the phrase allows a meaning that includes at least one of any one of the items, and/or at least one of any combination of the items. By way of example, the phrases "at least one of A, B, and C" or "at least one of A, B, or C" each refer to only A, only B, or only C; and/or any combination of A, B, and C. In instances where it is intended that a selection be of "at least one of each of A, B, and C," or alternatively, "at least one of A, at least one of B, and at least one of C," it is expressly described as such.

One general aspect includes a method for processing digital image data using a computing device, which comprises receiving a first image. The first image comprises a first region. The method further comprises generating a first confidence value using a first model. The first confidence value is associated with a first pixel of the first region, the first confidence value is associated with a first probability of the first region being a skin region. The method further comprises performing a first process at the first region based on the first confidence value.

Implementations may include one or more of the following features. The method further comprises generating a first mask using a first color model and the first image, generating a second mask using a second color model and the first image, and generating a third mask using a third color model and the first image. The first model is generated using at least one of the first color model, the second color model, or the third color model. The first confidence value is determined using at least the first mask, the second mask, and the third mask. The method further comprises generating a fourth mask using the first mask, the second mask, and the third mask based on a predetermined set of rules. The first model is generated using the fourth mask. The first process comprises a sharpening process at a level of sharpening associated with the first confidence value. The first pixel is characterized by a first color. The method further comprises receiving a color vision deficiency (CVD) profile from a user, determining a second color for the first pixel based on the CVD profile, and determining a third color for the first pixel by blending the first color with the second color based on the first confidence value. The first confidence value is less than or equal to 255. The method further comprises inputting the first confidence value and the first image into a second model to determine a first output for the first pixel. The first confidence value is associated with a second confidence value of a second pixel, and the second pixel is in a predetermined vicinity of the first pixel. The first output is determined based at least on the first confidence value and the second confidence value.

According to another embodiment, the subject technology provides an apparatus, which comprises a communication interface configured to receive a first image. The first image comprises a first region. The apparatus further comprises a memory coupled to the communication interface. The memory is configured to store the first image. The apparatus further comprises a processor coupled to the memory. The processor is configured to generate a first mask using a first color model and the first image. The processor is further configured to generate a second mask using a second color model and the first image. The processor is further configured to generate a first confidence value using at least the first mask and the second mask. The first confidence value is associated with a first pixel of the first region, the first confidence value is associated with a first probability of the first region being a skin region. The processor is further configured to perform a first process at the first region based on the first confidence value.

Implementations may include one or more of the following features. The apparatus further comprises a display configured to display the first region. The apparatus further comprises a user interface configured to receive a color vision deficiency (CVD) profile from a user. The processor comprises a central processing unit (CPU) and a graphics processing unit (GPU). The processor is further configured to generate a third mask using a third color model and the first image. The first confidence value is determined using at least the first mask, the second mask, and the third mask. The processor is further configured to generate a fourth mask by combining the first mask, the second mask, and the third mask based on a predetermined set of rules. The fourth mask comprises a grayscale image.

According to yet another embodiment, the subject technology provides a method for processing digital image data using a computing device, which comprises receiving a first image, the first image comprising a first region. The method further comprises generating a first mask using a first color model and the first image. The method further comprises generating a second mask using a second color model and the first image. The method further comprises generating a third mask using a third color model and the first image. The method further comprises generating a first confidence value using at least the first mask, the second mask, and the third mask. The first confidence value is associated with a first pixel of the first region, the first confidence value is associated with a first probability of the first region being a skin region. The method further comprises generating a fourth mask by combining the first mask, the second mask, and the third mask based on a predetermined set of rules. the first mask comprises a binary mask.

The first confidence value is less than or equal to 255.

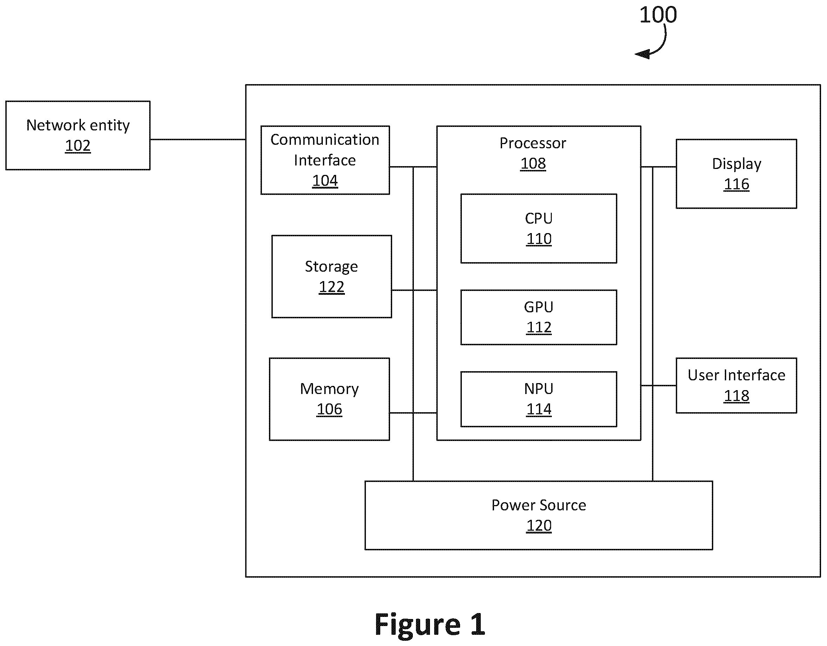

Figure 1 is a simplified diagram illustrating computing device 100 for video and image processing according to embodiments of the subject technology. This diagram is merely an example, which should not unduly limit the scope of the claims. One of ordinary skill in the art would recognize many variations, alternatives, and modifications.

A computing device capable of utilizing visual information associated with skin tones to generate an enhanced visual output is provided. In various embodiments, computing device 100 is configured to send and/or receive image and video data from a connected communication network or other networks. For example, computing device 100 receives an input image from an image source (e.g., network entity 102 or storage 122). The term "network entity" may refer to any device, platform, service, or infrastructure component that participates in the sending, receiving, storing, or processing of data over a network. This includes a wide array of entities that can interact with computing device 100 to provide data inputs or destinations for data outputs. Depending on the implementations, network entity 102 may be, without limitation, image-capturing devices, data storage services, streaming platforms, content sharing platforms, content delivery services, video telecommunication services, imaging devices, social networking platforms, gaming applications and services, mobile applications and services, and/or the like.

In various implementations, the input image includes an image stream or a video stream, which may be processed for enhanced skin detection and image quality enhancement. Depending on the application, the image or video data may be processed in real-time or near real-time. For instance, "real-time" processing may refer to the system's capability to produce output (e.g., image or video output) immediately as the data is captured, such as within a timeframe of milliseconds to a few seconds, ensuring that the output is produced with minimal latency that is imperceptible to users. The term "near real-time" may refer to processing that occurs swiftly after data capture, such as with a delay of a few seconds to a few minutes.

As shown, computing device 100 includes, without limitation, at least one of a communication interface 104, a memory 106, a power source 120, a display 116, a user interface 118, a processor 108, a storage 122, and/or the like. Computing device 100 may be implemented in hardware, software, or a combination of both. For example, computing device 100 may be, for example, servers, personal computers, smartphones, mobile devices, network servers, content servers, computer tablets, digital cameras, or any other processing devices.

In some instances, processor 108 may communicatively be coupled (e.g., via a bus, via wired connectors, or via electrical pathways (e.g., traces and/or pads, etc.) of printed circuit boards ("PCBs") or integrated circuits ("ICs"), and/or the like) to each of one or more of the communication interface 104, memory 106, power source 120, display 116, and/or user interface 118, storage 122, and/or the like.

In various implementations, communication interface 104 is configured to receive the input image from network entity 102 and permit data to be exchanged with network entity 102. The term "communication interface" may refer to hardware and/or software components that allow for the transmission and reception of data between the computing device and external sources or networks. For example, communication interface 104 includes, without limitation, a modem, a network card (wireless or wired), an infrared (IR) communication device, a wireless communication device, and/or chipset (such as a Bluetooth device, a WiFi device, a WiMax device, a WWAN device, a Z-Wave device, a ZigBee device, cellular communication facilities, etc.), and/or a low-power wireless device. In some examples, the input image includes a first region.

In some examples, communication interface 104 may be configured to receive a first image (e.g., the input image). It is to be appreciated that the first image may include a broad spectrum of visual data, including but not limited to, static images, a sequence of images or frames that constitute a video stream, and/or the like. The first image may include one or more regions of interest (e.g., a first region). For instance, in facial recognition applications, the first region may correspond to the facial area within a frame. Processor 108 may be configured to apply advanced algorithms (e.g., skin tone enhancement, feature accentuation, or artifact reduction) to the first region, utilizing the visual information to generate an enhanced output, as will be described in further detail below.

According to some embodiments, processor 108 is configured to take the input image as an input to perform an image processing operation to generate an enhanced image output. For instance, the term "processor" may refer to an electronic component or group of components that execute various types of computational tasks within a computing device. In some embodiments, processor 108 includes various types of processing units, such as a central processing unit (CPU) 110, a graphics processing unit (GPU) 112, and/or a neural processing unit (NPU) 114.

Different types of processing units are optimized for different types of computations. For example, the term "central processing unit" may refer to the primary component of a computing device that performs the majority of processing tasks, such as arithmetic calculations, logic operations, controlling other components, handling input/output operations, and/or the like. In various implementations, CPU 110 manages various types of general computations, such as directing the flow of data between the modules of computing device 100, executing instruction sets for skin detection algorithms, handling system operations, and/or the like.

In some examples, the term "graphics processing unit" may refer to a specialized electronic component designed to accelerate computer graphics and image processing. GPU 112 may be specifically designed to handle graphics and image processing tasks, providing accelerated computing power for high-resolution and complex image manipulations.

The term "neural processing unit" may refer to a specialized electronic component designed to accelerate the execution of neural networks. For instance, NPU 114 is optimized for running machine learning models, such as convolutional neural networks (CNNs) or deep neural networks (DNNs), which are central to adaptive skin detection and processing algorithms. In certain embodiments, NPU 114 is configured to perform advanced image processing techniques, such as adaptive smoothing and edge enhancement based on the skin confidence map. NPU 114 enables computing device 100 to learn from data, thereby continuously refining its skin detection and image enhancement algorithms over time. It is to be appreciated that processor 108 may be configured as a multi-core processor with one or more processing units, each capable of independently executing program instructions. This arrangement allows for efficient parallel processing and reduced overall power consumption, providing the capability to handle the demanding requirements of real-time or near real-time image and video processing for applications ranging from consumer-level photo editing to professional-grade media production.

In various embodiments, computing device 100 includes one or more storage devices including, for example, storage 122 and/or memory 106. For example, the term "memory" may refer to a hardware component used to store data temporarily during the operation of the computing device. Memory 106 may include, without limitation, random-access memory (RAM), dynamic random-access memory (DRAM), flash memory, static random-access memory (SRAM), and/or the like. The term "storage" may refer to hardware components that permanently store data. Storage 122 may include, without limitation, local and/or network-accessible storage, a disk drive, a drive array, an optical storage device, a solid-state storage device such as a read-only memory (ROM), which can be programmable, flash-updateable, and/or the like. In various embodiments, storage 122 may be implemented as a part of the processor 108 in a system-on-chip (SoC) arrangement. In some instances, the input image may be temporarily stored in memory 106 for further processing, and executable instructions (e.g., skin detection algorithms, image enhancement algorithms, and/or the like) may be stored in storage 122.

Power source 120 may be coupled to processor 108 to provide processing power to assist with processing loads experienced in computing device 100. Power source 120 may include, for example, a battery and/or a wired power source. As explained above, one or more processing units of processor 108 may retrieve and execute instructions simultaneously for energy-efficient operation.

In certain embodiments, computing device 100 further includes display 116, which may be configured to output the visually enhanced image content. The term "display" may refer to a device for visual output that presents images, videos, or any other graphical content to the user. For instance, display 116 may include, without limitation, a liquid crystal display (LCD), a light-emitting diode (LED) screen, an organic LED (OLED) display, a flat panel, a solid-state display, and/or the like.

In some examples, computing device 100 may additionally include or be in communication with user interface 118. The term "user interface" may refer to hardware and/or software components that allow a user to interact with a computing device. User interface 118 may include, without limitation, a mouse, a keyboard, a remote control, one or more sensors, and/or the like. In various implementations, user interface 118 may be configured to receive user-specific information, such as a CVD profile, enabling computing device 100 to customize its image processing algorithms to meet individual visual requirements. When users input their distinct CVD parameters, processor 108 may adjust the image output specifically for their visual perception needs. This ensures that the images processed by computing device 100 are not only enhanced but also accessible and perceived accurately according to each user's unique vision.

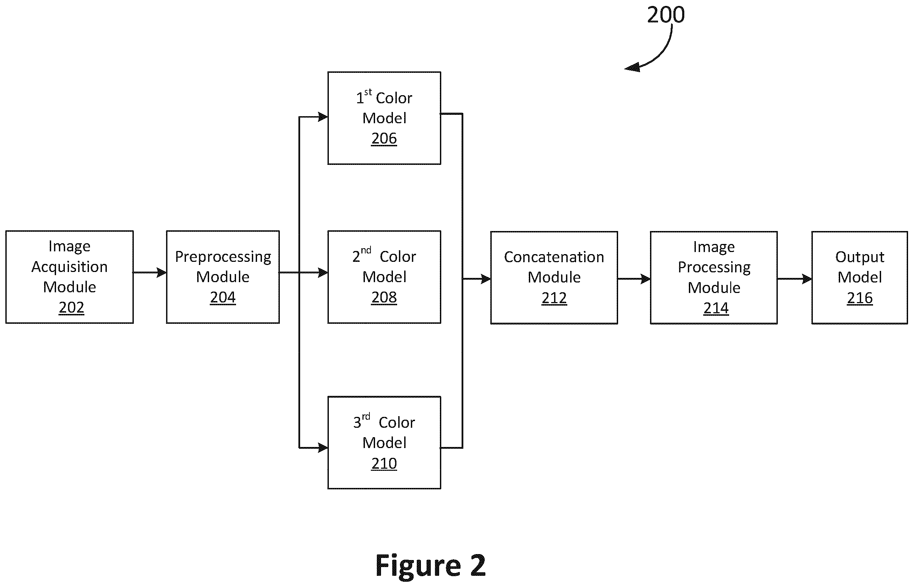

Figure 2 is a simplified diagram illustrating a system 200 for video and image processing according to embodiments of the subject technology. This diagram is merely an example, which should not unduly limit the scope of the claims. One of ordinary skill in the art would recognize many variations, alternatives, and modifications.

In various implementations, image acquisition module 202 receives and/or captures a first image from an image source (e.g., network entity 102 or storage 122 of Figure 1). The first image may include a broad spectrum of visual data including, but not limited to, static images, a sequence of images or frames that constitute a video stream, and/or the like. For instance, the first image may be captured by digital cameras, surveillance systems, or retrieved from archival footage. The first image may include a sequence of frames for video or a single frame for still images, associated with one or more subjects (e.g., individuals, groups, etc.). In some examples, the first image may include one or more regions of interest (e.g., a first region). For instance, in facial recognition applications, the first region may correspond to the facial area within a frame. The first image may subsequently be processed through a series of modules of system 200 designed to enhance the visual quality of skin tones and features.

Following image acquisition, preprocessing module 204 may perform preliminary adjustments to the first image. These adjustments may include tasks such as normalization, noise reduction, resizing, or color correction, which prepare the first image for subsequent processing.

In some embodiments, one or more color models (e.g., first color model 206, second color model 208, and/or third color model 210) may be applied to the preprocessed image. The term "color model" may refer to a mathematical system that is used to represent and manipulate color information within a digital context. For instance, this representation involves expressing color data as numerical tuples, such as three or four values, corresponding to the intensities of various light and spectral components. Each color model defines a specific color space through its parameters, encompassing the representable color gamut within the system. For instance, applying a color model involves the analysis of image data based on the specific parameters defined by the color model. These parameters dictate how colors are encoded and interpreted, providing a framework for color translation and manipulation within the image processing pipeline.

In various examples, the deployment of each color model is designed to analyze different attributes of the visual data, allowing for targeted extraction of features that are relevant to skin detection. By applying distinct color models, system 200 is capable of generating masks, wherein pixels are evaluated and marked to create a confidence map that distinguishes skin from non-skin areas within the image. For example, the term "mask" may refer to a digital filter that is used to categorize pixels within an image. Depending on the implementation, one or more color models may include, without limitation, RGB (Red, Green, Blue), HSV (Hue, Saturation, Value), HSL (Hue, Saturation, Lightness), YUV (Luminance, Chrominance), CMYK (Cyan, Magenta, Yellow, Key/Black), YCbCr (Luminance, Blue-difference Chroma, Red-difference Chroma), Adobe RGB, and/or the like.

According to some embodiments, first color model 206 may be used to obtain a first mask. For instance, first color model 206 may include an RGB model. The RGB model may be based on an additive color process, where colors are produced by combining varying intensities of red, green, and blue light. Each color within the RGB model is represented as a combination of the three channels, corresponding to red, green, and blue light intensities. For the purpose of skin detection, the RGB model may be employed to analyze the color information of each pixel in the first image. The model's parameters are tuned to detect the hues commonly associated with human skin by setting specific ranges for red, green, and blue intensities that reflect the skin tones. These parameters may be analyzed to identify pixels within a predefined skin tone range, generating the first mask that highlights areas of potential skin presence.

In some examples, the first mask may include a binary mask. The term "binary mask" may refer to a data array that assigns a "1" or "0" to image pixels to differentiate between areas of interest and the background based on specific criteria. For instance, in the first mask, pixels with a value of "1" are considered potential skin pixels, while pixels with a value of "0" indicate non-skin regions.

Depending on the implementation, one or more RGB masks may be generated based on a set of threshold conditions for the red, green, and blue channels. In some examples, a first RGB mask may be generated by applying a first set of criteria, which may include requiring the red value (R) to be within a specific range (e.g., 20 < R < 255), the green value (G) to be within a certain range (e.g., 40 < G < 255), and the blue value (B) to be within a certain range (e.g., 65 < B < 255). In some cases, additional constraints may be applied to the difference between the red and green values (e.g., 8 < (R-G) < 90 or 12 < (R-G) < 112) to further refine the mask and exclude non-skin pixels with similar color components.

Similarly, a second RGB mask may be generated by applying a second set of criteria, tailored to detect subtle variations in skin tone that may be attributed to different lighting conditions or ethnic backgrounds. For instance, the second set of criteria may further refine the red channel values used in the first mask (e.g., 220<R<255), such as targeting darker or lighter skin tones. Additionally, the red-green differential constraints may be adjusted to cover a wider range of color variations commonly found in human skin (e.g., 0<(R-G)<90 or 0<(R-G)<112).

In various embodiments, a final RGB mask (e.g., the first mask) can be generated by combining the individual RGB masks (e.g., the first and second RGB masks) through a logical OR operation. This combined mask effectively identifies potential skin regions by utilizing both sets of threshold conditions. Pixels satisfying the potential skin pixel criteria in either the first or second RGB mask are assigned a value of "1" in the final mask, indicating a high probability of skin presence. Employing multiple masks with varying thresholds enables system 200 to capture a broad spectrum of skin tones. Such an approach accounts for variations in skin tone intensity and lighting conditions, thus enhancing the precision of skin detection across diverse imaging scenarios. In some cases, the RGB color space may be utilized to convert to other color models or spaces that offer enhanced color differentiation for precise skin detection in various image processing applications.

According to some embodiments, second color model 208 may be used to obtain a second mask. For instance, second color model 208 may include a Kullback-Leibler (KL) model. The KL model may utilize the KL divergence to quantify the difference between two probability distributions, which may be used to detect variations in color distributions within the first image that are indicative of skin presence. For example, the KL model may be applied in skin detection by comparing the probability distribution of a pixel's color components (e.g., red, green, and blue) in the first image with a reference distribution representative of human skin tones. The KL divergence between these two distributions indicates how likely a pixel's color deviates from the expected range of skin tones.

In various implementations, the KL model utilizes a transformation matrix to convert the RGB pixel components (red, green, and blue) into a new color space with three derived values, K1, K2, and K3 (which may be referred to as "KL coordinates"). The transformation matrix may be represented as follows:

$$\begin{pmatrix} K_1 \\ K_2 \\ K_3 \end{pmatrix} = \begin{pmatrix} 0.666 & 0.547 & 0.507 \\ -0.709 & 0.255 & 0.657 \\ 0.230 & -0.797 & 0.558 \end{pmatrix} \begin{pmatrix} R \\ G \\ B \end{pmatrix}$$

A KL mask (e.g., the second mask) may then be generated by comparing the values of K1, K2, and K3 color components for each pixel against a set of predetermined thresholds. The second mask may include a binary mask. For example, pixels where all three KL coordinates fall within specific ranges (e.g., 110.2 < K1 < 410, -61.3 < K2 < 32.9, -18.8 < K3 < 26) are assigned a value of "1" in the mask. These thresholds are established to capture color components likely associated with human skin tones based on the KL divergence analysis. Conversely, pixels with KL coordinates outside these threshold ranges are assigned a value of "0" in the mask, indicating a lower probability of skin presence.

According to some embodiments, third color model 210 may be used to obtain a third mask. For instance, third color model 210 may include a YCbCr model. The YCbCr model may utilize the YCbCr color space, which separates the image data into luminance (Y) and chrominance (Cr and Cb) components. The luminance component (Y) represents the brightness of a pixel, while the chrominance components (Cr and Cb) encode the color information relative to a reference level. This separation allows the YCbCr model to analyze the first image by focusing on the differences in color and brightness, which is beneficial in distinguishing skin tones from backgrounds or non-skin elements under various lighting conditions.

Depending on the implementation, the YCbCr model may generate one or more YCbCr masks (e.g., the third mask). These YCbCr masks may represent varying confidence levels in identifying skin presence at particular pixels, based on the YCbCr color space. For instance, the third mask may include a binary mask. In some examples, a first YCbCr mask may be generated to identify regions with a reasonable probability of being skin. It considers a broad range of chrominance (Cr and Cb) values that encompass various skin tones while also incorporating luminance (Y) to reflect differences in skin brightness. In some embodiments, the generation of the first YCbCr mask may involve two sets of criteria depending on the pixel's luminance (Y) value. For instance, for brighter pixels (e.g., Y > 128), a first set of criteria may be applied, which includes at least one of the following

conditions:

- 

$$-17<(Cr+Cb)$$

- 

$$(6Cr+Cb)<250$$

- 

$$35<=(Cr-2.5Cb+2.5Y/16)$$

- 

$$(3Cr+4Cb-Y/4)<=16$$

- 

$$-4<=(Cr+0.5Cb)$$

For darker pixels (e.g., Y<=128), a second set of criteria may be applied, which includes at least one of the following conditions:

- 

$$-17<(Cr+Cb)$$

- 

$$(6Cr+Cb)<250$$

- 

$$12<=(Cr-2.5Cb)$$

- 

$$(3Cr+4Cb)<=58$$

- 

$$6<=(Cr-Y/32)$$

- 

$$-8<=(Cr+0.5Cb-Y/32)$$

In some implementations, a second YCbCr mask may be generated to identify pixels with a high probability of being skin. For example, the second YCbCr mask may employ stricter criteria within the YCbCr color space compared to the first YCbCr mask. By focusing on a narrower range of chrominance values (Cr and Cb) that are highly characteristic of human skin tones, the second YCbCr mask can achieve a more precise detection of skin regions within the first image. For instance, for brighter pixels (e.g., Y > 128), one or more of the following conditions may be applied:

- 

$$-48<(Cr+2Cb)$$

- 

$$-17<(Cr+Cb)$$

- 

$$-128<(Cr+4Cb)$$

- 

$$(6Cr+Cb)<250$$

- 

$$35<=(Cr-2.5Cb+2.5Y/16)$$

- 

$$(3Cr+4Cb-Y/4)<=16$$

- 

$$-2<=(Cr+0.5Cb)$$

For darker pixels (e.g., Y<=128), one or more of the following conditions may be applied:

- 

$$-48<(Cr+2Cb)$$

- 

$$-17<(Cr+Cb)$$

- 

$$-128<(Cr+4Cb)$$

-

$$(6Cr+Cb)<250$$

- 

$$15<=(Cr-2.5Cb)$$

- 

$$(3Cr+4Cb)<=48$$

- 

$$12<=(Cr-Y/32)$$

- 

$$-8<=(Cr+0.5Cb-Y/32)$$

In various implementations, concatenation module 212 may be configured to synthesize the masks generated by first color model 206, second color model 208, and third color model 210 into a composite mask (e.g., a fourth mask). For example, the fourth mask includes a grayscale image. The term "grayscale image" may refer to an image composed of varying shades of gray, in which each pixel represents an intensity value on a scale of black to white. In some embodiments, the grayscale image may serve as a skin confidence map, where each pixel is assigned a confidence value indicating the likelihood of skin presence. The term "confidence value" may refer to a numerical representation of the probability that a given pixel or group of pixels corresponds to a skin region within an image. In an example, the fourth mask may include a first confidence value, which may be associated with a first pixel of the first region. The first confidence value may be associated with a first probability of the first region being a skin region. For instance, the first confidence value ranges from 0 to 255, where a value of 0 indicates the lowest likelihood (e.g., a non-skin region), and a value of 255 denotes the highest likelihood of skin presence.

In some embodiments, the fourth mask may be generated by combining the first mask, the second mask, and the third mask (e.g., the first YCbCr mask and/or the second YCbCr mask) based on a predetermined set of rules. In certain examples, the predetermined set of rules may be based on the degree of agreement among the individual masks, each representing different criteria and perspectives of skin detection. For instance, a pixel that meets the skin-region criteria in all four binary masks is accorded the highest confidence value of "255," indicating a high probability of skin presence. If three out of four maks (e.g., the first mask, the second maks, and the first YCbCr mask) identify a skin pixel, such a pixel is assigned a value of "220." If any two of the masks align on skin identification for a pixel, that pixel is accorded a value of "120," indicating moderate confidence in its classification as a skin region. Any remaining pixel not captured by the previous conditions is assigned a value of "0," indicating no skin presence.

It is to be appreciated that incorporating multiple binary masks into a single skin confidence map enhances skin detection accuracy by capturing a comprehensive array of skin-tone attributes and variances. This composite mask not only improves the precision of skin detection but also demonstrates adaptability to different skin tones, lighting conditions, and ethnic backgrounds. Additionally, it boosts processing efficiency by reducing calculation overhead, enabling the swift identification of skin regions with minimal latency-enhancing the system's capability to process videos in real-time or near real-time.

In certain implementations, image processing module 214 may utilize the fourth mask (e.g., the skin confidence map) to execute image enhancements tailored to skin regions. For instance, image processing module 214 may perform a first process at the first region based on the first confidence value. The first process may include one or more image enhancement techniques, such as sharpening, contrast adjustment, saturation modification, texture smoothing, color correction, and/or the like. For example, the first process comprises a sharpening process, where the level of sharpening is associated with the first confidence value. In other words, image processing module 214 may adjust the intensity of the sharpening effect based on the confidence value assigned to each pixel. The term "sharpening" may refer to a digital image processing technique that enhances image clarity by emphasizing edges and transitions between areas of contrasting colors. This process can improve the perception of detail and definition within an image.

In some examples, image processing module 214 may apply different levels of sharpening to image regions according to the confidence values. Regions with higher confidence values-indicating a greater likelihood of skin presence-may undergo less sharpening to avoid artificiality. Regions with lower confidence values-suggesting a lower likelihood of skin presence-may receive a stronger degree of sharpening to enhance details and clarity. In video processing applications, image processing module 214 may dynamically adjust sharpening levels frame by frame. This ensures consistency and accuracy in identifying skin regions across varying scenes and lighting conditions, providing a seamless viewing experience.

According to some embodiments, image processing module 214 may implement a weighted addition technique, merging sharpened and unsharpened regions in line with the skin confidence mask. This process employs each mask value as a weight factor, guiding the blend of sharpened and original image data for optimal visual fidelity.

Depending on the application, image processing module 214 may be configured to improve image accessibility for individuals with CVD. The term "color vision deficiency" may refer to a visual condition that affects how people perceive colors. Individuals with CVD might have difficulty distinguishing between certain colors, often experiencing difficulties with red-green or blue-yellow differentiation. To address this, accessibility processing for CVD may be implemented, allowing users to specify their type of colorblindness and its severity through a user interface (e.g., user interface 118 of Figure 1). This user input informs the processing of the image or video to remap colors that are challenging for a CVD viewer to differentiate into more distinguishable hues.

However, this global adjustment can inadvertently alter the appearance of skin tones, leading to unnatural results that are easily detectable due to the human sensitivity to facial color discrepancies. To mitigate this effect, image processing module 214 may utilize the skin confidence map to guide color mapping adjustments specifically tailored to CVD users. For example, image processing module 214 may control the degree of color mapping based on the confidence values provided by the skin confidence map. In areas where the confidence map indicates high confidence, the original skin colors may be maintained to a great extent, ensuring that skin regions retain their natural tones. Conversely, in areas with low confidence values or outside skin regions, the colors are modified more significantly to enhance contrast and visibility.

In an example, the first pixel is characterized by a first color. Image processing module 214 may determine a second color for the first pixel based on a CVD profile of a user and then determine a third color for the first pixel by blending the first color with the second color based on the first confidence value. This selective mapping ensures that while the overall color distinction is enhanced for accessibility, the integrity of skin tones remains largely unaffected, maintaining a realistic appearance to all viewers, regardless of their CVD status.

In various examples, a first model may be used to perform skin detection and/or image and video processing. The term "model" may refer to a computational framework or algorithmic structure designed to analyze and interpret data for specific applications. For instance, the first model may employ various techniques, such as machine learning, statistical analysis, pattern recognition, and advanced computational algorithms to interpret and synthesize data for skin detection. In some examples, the first model may include a machine learning (ML) model. The term "machine learning model" may refer to a computational algorithm that, based on the analysis of data, learns patterns and makes decisions with minimal human intervention. The first model may include various configurations, such as CNNs, DNNs, support vector machines (SVMs), recurrent neural networks (RNNs), generative adversarial networks (GANs), random forests, deep learning networks, statistical models, and/or the like.

In various implementations, the first model may synthesize information obtained from one or more color models into a unified representation of skin presence probabilities. For instance, the first model may be generated using at least one of the first color model, the second color model, or the third color model. The first model may integrate data from one or more color spaces to enhance the accuracy of skin detection. This integration may involve aggregating the outputs from each color model and applying weighted factors to each depending on their reliability and relevance in various imaging conditions. In some examples, the first model may use the combined data to generate the skin confidence map, where each pixel's likelihood of being part of a skin region is computed based on the aggregated values from multiple color models. For instance, the first model may be configured to generate a first confidence value associated with a first pixel of the first region. The first confidence value is associated with a first probability of the first region being a skin region.

In some examples, the fourth mask-which may be a composite mask derived from individual masks corresponding to each color model-can be used to generate the first model. The first model may use this composite mask to learn how to weigh the input from each color model dynamically, adjusting its parameters through training iterations to optimize skin detection accuracy.

In some cases, to further refine the skin detection output and reduce noise, the composite skin mask may undergo a smoothing process at image processing module 214. The mask may be taken through a smoothing filter to smooth out sudden anomalies. This smoothing process helps to even out the confidence values across adjacent pixels, thereby reducing isolated errors and enhancing the overall coherence of the detected skin regions.

According to some embodiments, image processing module 214 may integrate a second model to refine the processes of skin detection and image enhancement. For example, the second model may include various configurations, such as CNNs, DNNs, SVMs, RNNs, GANs, random forests, deep learning networks, statistical models, and/or the like.

In certain implementations, the second model may utilize the skin confidence map as a part of the training dataset. The second model may be trained to further refine the image processing performed by image processing module 214. For instance, the second model learns to use the confidence values and their spatial distributions as input features to generate refined outputs. For example, a CNN within the second model may be trained to apply different processing techniques to areas of varying confidence levels, ensuring that each region is enhanced in a manner that respects the underlying skin presence probability. It is to be appreciated that the second model may be trained to interpret the confidence values, not only at a pixel level but also across neighboring pixel regions, effectively utilizing the spatial context of each pixel's vicinity. This contextual information allows the model to differentiate between skin and non-skin regions with enhanced clarity, such as along edges where abrupt changes in confidence values may occur.

In an example, the first confidence value and the first image may be input to the second model to determine a first output for the first pixel. The first confidence value is associated with a second confidence value of a second pixel, which is in a predetermined vicinity of the first pixel. For instance, the term "vicinity" may refer to the neighboring pixels within a certain radius or a defined pixel grid surrounding a pixel. For example, a predetermined vicinity may include a 3x3 or 5x5 pixel grid surrounding the pixel of interest. The first output may be determined based at least on the first confidence value and the second confidence value. By analyzing confidence values within the defined vicinity, the second model can effectively interpret how the confidence value of a single pixel correlates with the values of its neighbors, providing a comprehensive understanding of the image structure. This contextual analysis is beneficial in edge processing or smoothing, as it ensures transitions between skin and non-skin regions are handled with enhanced finesse, avoiding harsh edges or unrealistic blending in the final image composition.

In video processing applications, the second model may be configured to analyze sequential frames to anticipate changes in lighting or movement, thereby continuously adjusting the skin detection and image enhancement algorithms to maintain consistency across frames. This constant adjustment can lead to a more natural and seamless viewing experience, which is beneficial in real-time applications such as live broadcasts or video calls.

In some embodiments, the second model may be employed to tailor image processing parameters for users with CVD, learning from user interactions to better accommodate individual preferences and enhancing the accessibility of the images or videos without compromising the natural appearance of skin tones. For example, based on user feedback, the model can fine-tune the strength of color mapping adjustments applied to non-skin regions while preserving the integrity of skin tones based on users' CVD profiles. This adaptive learning approach ensures a more personalized viewing experience for users with different color vision needs.

In various implementations, output module 216 functions as the final stage in the image processing pipeline, where the processed image or video data is prepared for display. For instance, output module 216 may include, without limitation, a liquid crystal display, a light-emitting diode screen, an organic LED display, a flat panel, a solid-state display, and/or the like.

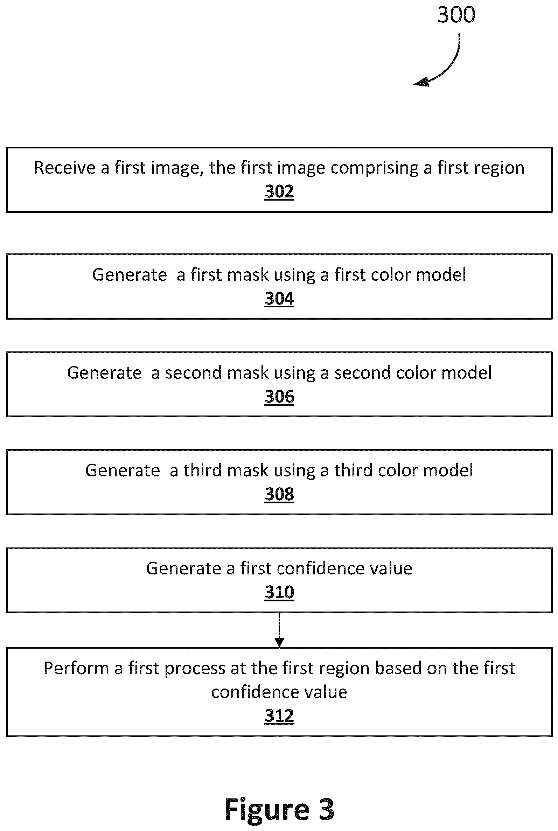

Figure 3 is a simplified flow diagram illustrating method 300 for video and image processing according to embodiments of the subject technology. This diagram is merely an example, which should not unduly limit the scope of the claims. One of ordinary skill in the art would recognize many variations, alternatives, and modifications. For example, one or more steps may be added, removed, repeated, modified, replaced, overlapped, and/or rearranged, and should not limit the scope of the claims.

At step 302, a first image is received. The first image may include a broad spectrum of visual data including, but not limited to, static images, a sequence of images or frames that constitute a video stream, and/or the like. For instance, the first image may be captured by digital cameras, surveillance systems, or retrieved from archival footage. The first image may include a sequence of frames for video or a single frame for still images, associated with one or more subjects (e.g., individuals, groups, etc.). In some examples, the first image may include one or more regions of interest (e.g., a first region). For instance, the first region may correspond to the facial area within a frame.

At step 304, a first mask is generated using a first color model. For instance, the first color model may include an RGB model. The RGB model may be employed to analyze the color information of each pixel in the first image. The model's parameters are tuned to detect the hues commonly associated with human skin by setting specific ranges for red, green, and blue intensities that reflect the skin tones. These parameters may be analyzed to identify pixels within a predefined skin tone range, generating the first mask that highlights areas of potential skin presence. In some cases, the first mask may include a binary mask, where pixels with a value of "1" are considered potential skin pixels, and pixels with a value of "0" indicate non-skin regions.

At step 306, a second mask is generated using a second color model. For instance, the second color model may include a Kullback-Leibler (KL) model. The KL model may utilize the KL divergence to quantify the difference between two probability distributions, which may be used to detect variations in color distributions within the first image that are indicative of skin presence. For example, the KL model may be applied in skin detection by comparing the probability distribution of a pixel's color components (e.g., red, green, and blue) in the first image with a reference distribution representative of human skin tones. The second mask (e.g., a KL mask) may include a binary mask, which represents a binary categorization of each pixel based on the KL divergence outcomes.

At step 308, a third mask is generated using a third color model. For instance, third color model 210 may include a YCbCr model. The YCbCr model may generate one or more YCbCr masks (e.g., the third mask). These YCbCr masks may represent varying confidence levels in identifying skin presence at particular pixels, based on the YCbCr color space.

At step 310, a first confidence value is generated. The first confidence value may be generated using the first mask, the second mask, and the third mask. For example, a fourth mask is generated by combining the first mask, the second mask, and the third mask based on a predetermined set of rules. The fourth mask may include the first confidence value, which may be associated with a first pixel of the first region. The first confidence value may be associated with a first probability of the first region being a skin region. The term "skin region" may refer to an area of an image that represents the skin of a subject within the image. For instance, the first confidence value ranges from 0 to 255, where a value of 0 indicates the lowest likelihood (e.g., a non-skin region), and a value of 255 denotes the highest likelihood of skin presence.

At step 312, a first process is performed at the first region based on the first confidence value. The first process may include one or more image enhancement processes, such as selective sharpening, where areas with higher confidence values indicative of skin receive minimal sharpening to preserve natural textures, while areas with lower confidence values receive a stronger degree of sharpening to enhance details and clarity. In some cases, the first process may also include dynamic color adjustment, softening or smoothing of high-confidence skin regions to reduce artifacts, and/or targeted color correction to ensure skin tones are rendered faithfully.

While the above is a full description of the specific embodiments, various modifications, alternative constructions, and equivalents may be used. Therefore, the above description and illustrations should not be taken as limiting the scope of the present invention which is defined by the appended claims.

**Claims**

1. A method for processing digital image data using a computing device comprising:

   receiving a first image, the first image comprising a first region;
   generating a first confidence value using a first model, the first confidence value being associated with a first pixel of the first region, the first confidence value being associated with a first probability of the first region being a skin region; and
   performing a first process at the first region based on the first confidence value.

2. The method of claim 1, further comprising:

   generating a first mask using a first color model and the first image;
   generating a second mask using a second color model and the first image; and
   generating a third mask using a third color model and the first image;
   wherein the first model is generated using at least one of the first color model, the second color model, or the third color model,
   in particular,
   wherein the first confidence value is determined using at least the first mask, the second mask, and the third mask.

3. The method of claim 2, further comprising generating a fourth mask using the first mask, the second mask, and the third mask based on a predetermined set of rules;
   wherein the first model is generated using the fourth mask.

4. The method of one of the previous claims, wherein the first process comprises a sharpening process at a level of sharpening associated with the first confidence value.

5. The method of one of the previous claims, wherein the first pixel is **characterized by** a first color, and the method further comprises:

   receiving a color vision deficiency (CVD) profile from a user;
   determining a second color for the first pixel based on the CVD profile; and
   determining a third color for the first pixel by blending the first color with the second color based on the first confidence value.

6. The method of one of the previous claims, wherein the first confidence value is less than or equal to 255.

7. The method of one of the previous claims, further comprising inputting the first confidence value and the first image into a second model to determine a first output for the first pixel, wherein the first confidence value is associated with a second confidence value of a second pixel, and the second pixel is in a predetermined vicinity of the first pixel,
   in particular,
   wherein the first output is determined based at least on the first confidence value and the second confidence value.

8. An apparatus comprising:

   a communication interface configured to receive a first image, the first image comprising a first region;
   a memory coupled to the communication interface, the memory being configured to store the first image;
   a processor coupled to the memory, the processor being configured to:

generate a first mask using a first color model and the first image;

generate a second mask using a second color model and the first image;

generate a first confidence value using at least the first mask and the second mask, the first confidence value being associated with a first pixel of the first region, the first confidence value being associated with a first probability of the first region being a skin region; and

perform a first process at the first region based on the first confidence value.

9. The apparatus of claim 8, further comprising a display configured to display the first region
and/or
further comprising a user interface configured to receive a color vision deficiency (CVD) profile from a user.

10. The apparatus of claims 8 or 9, wherein the processor comprises a central processing unit (CPU) and a graphics processing unit (GPU).

11. The apparatus of one of claims 8 to 10, wherein:

the processor is further configured to generate a third mask using a third color model and the first image; and

the first confidence value is determined using at least the first mask, the second mask, and the third mask.

12. The apparatus of claim 11, wherein the processor is further configured to generate a fourth mask by combining the first mask, the second mask, and the third mask based on a predetermined set of rules, in particular,

wherein the fourth mask comprises a grayscale image.

13. A method for processing digital image data using a computing device comprising:

receiving a first image, the first image comprising a first region;

generating a first mask using a first color model and the first image;

generating a second mask using a second color model and the first image;

generating a third mask using a third color model and the first image; and

generating a first confidence value using at least the first mask, the second mask, and the third mask, the first confidence value being associated with a first pixel of the first region, the first confidence value being associated with a first probability of the first region being a skin region.

14. The method of claim 13, further comprising generating a fourth mask by combining the first mask, the second mask, and the third mask based on a predetermined set of rules.

15. The method of claims 13 or 14, wherein the first mask comprises a binary mask
and/or
wherein the first confidence value is less than or equal to 255.

**Figure 1**

200

Image
Acquisition
Module
202

Preprocessing
Module
204

1st Color
Model
206

2nd Color
Model
208

3rd Color
Model
210

Concatenation
Module
212

Image
Processing
Module
214

Output
Model
216

**Figure 2**

300

Receive a first image, the first image comprising a first region
**302**

Generate  a first mask using a first color model
**304**

Generate  a second mask using a second color model
**306**

Generate  a third mask using a third color model
**308**

Generate a first confidence value
**310**

Perform a first process at the first region based on the first confidence value
**312**

# Figure 3

Europäisches
Patentamt

European
Patent Office

Office européen
des brevets

## EUROPEAN SEARCH REPORT

**Application Number**

EP 25 17 3979

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/298704 A1 (NACHLIELI HILA [IL] ET AL) 4 December 2008 (2008-12-04) | 1-4,6-15 | INV.<br>G06V10/25 |
| A | * paragraph [0032] - paragraph [0056]; figure 1 * | 5 | G06V10/20<br>G06V40/16 |
| | ----- | | |
| A | US 8 406 482 B1 (CHIEN JEN-CHAN [US] ET AL) 26 March 2013 (2013-03-26)<br>* claim 1 * | 1,8,13 | |
| | ----- | | |
| A | WO 2007/022413 A2 (QUALCOMM INC [US]; QUAN SHUXUE [US] ET AL.)<br>22 February 2007 (2007-02-22)<br>* paragraph [0095] - paragraph [0097] * | 1,8,13 | |
| | ----- | | |

**TECHNICAL FIELDS SEARCHED (IPC)**

G06V

The present search report has been drawn up for all claims

| Place of search | Date of completion of the search | Examiner |
|---|---|---|
| The Hague | 22 August 2025 | Nowbakht Irani, Ali |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 17 3979

This annex lists the patent family members relating to the patent documents cited in the above-mentioned European search report.
The members are as contained in the European Patent Office EDP file on
The European Patent Office is in no way liable for these particulars which are merely given for the purpose of information.

22-08-2025

| Patent document cited in search report | | Publication date | Patent family member(s) | | Publication date |
|---|---|---|---|---|---|
| US 2008298704 | A1 | 04-12-2008 | EP | 2176830 A1 | 21-04-2010 |
| | | | US | 2008298704 A1 | 04-12-2008 |
| | | | WO | 2008153702 A1 | 18-12-2008 |
| US 8406482 | B1 | 26-03-2013 | NONE | | |
| WO 2007022413 | A2 | 22-02-2007 | CN | 101288103 A | 15-10-2008 |
| | | | EP | 1915737 A2 | 30-04-2008 |
| | | | JP | 5496509 B2 | 21-05-2014 |
| | | | JP | 2009505107 A | 05-02-2009 |
| | | | JP | 2012168181 A | 06-09-2012 |
| | | | KR | 20080045175 A | 22-05-2008 |
| | | | US | 2007043527 A1 | 22-02-2007 |
| | | | US | 2012176507 A1 | 12-07-2012 |
| | | | WO | 2007022413 A2 | 22-02-2007 |

EPO FORM P0459

For more details about this annex : see Official Journal of the European Patent Office, No. 12/82